# EUROPEAN PATENT APPLICATION

(11) **EP 3 925 990 A1**
(43) Date of publication of application: **22.12.2021**
(21) Application number: 20755231.6
(22) Date of filing: 12.02.2020
(51) Int. Cl.: C08F 220/34, C08F 230/02, C09D 133/14

(54) **METHOD FOR PRODUCING POLYMER COMPATIBLE WITH BIOMATERIALS**

(30) Priority: 12.02.2019 JP 2019022902
(71) Applicant: Nissan Chemical Corporation, Tokyo 103-6119 (JP)
(72) Inventor: HIROI Miya, Funabashi-shi, Chiba 274-0052 (JP); HIROI Yoshiomi, Funabashi-shi, Chiba 274-0052 (JP); SUZUKI Kohei, Funabashi-shi, Chiba 274-0052 (JP); SHOJI Takeaki, Funabashi-shi, Chiba 274-8507 (JP); ABE Natsuki, Shiraoka-shi, Saitama 349-0294 (JP); NAKAJIMA Hiroyuki, Shiraoka-shi, Saitama 349-0294 (JP)
(74) Representative: dompatent von Kreisler Selting Werner - Partnerschaft von Patent- und Rechtsanwälten mbB
(86) International application number: PCT/JP2020/005331
(87) International publication number: WO 2020/166605

(57) **Abstract**

This is to provide a method for producing a copolymer-containing varnish using a monomer having a specific unsaturated bond which does not cause the problem of gelation, a method for producing a composition, a method for producing a coating film, preferably a method for producing a coating film compatible with biomaterials (for example, having an adhesion suppressing ability to biomaterials and a cell culture promoting property).

The method for producing a copolymer-containing varnish which comprises a step of copolymerizing a monomer mixture containing compounds represented by the following formula (1) and the formula (2): 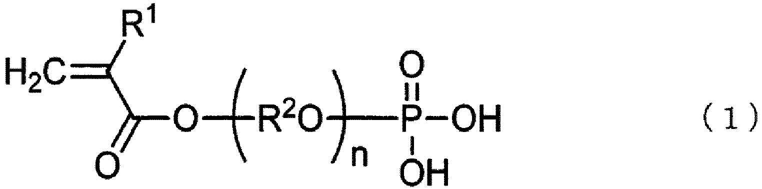 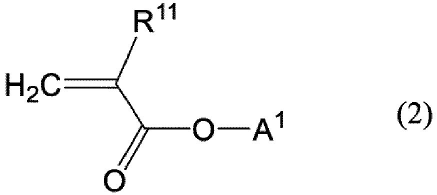 (in the formula (1), R¹ represents a hydrogen atom or a methyl group, R² represents an alkylene group having 1 to 6 carbon atoms, n represents an integer of 1 to 30, in the formula (2), R¹¹ represents a hydrogen atom or a methyl group, and A¹ represents a monovalent organic group having a cationic property) in a solvent for polymerization, in the presence of a radical polymerization initiator, wherein % by mass of the compound represented by the formula (1) in a phosphorus-containing compound contained in the monomer mixture is 70% by mass or more.

## Description

### TECHNICAL FIELD

The present invention relates to a method for producing a copolymer-containing varnish which does not cause the problem of gelation, a method for producing a composition for forming a coating film, a method for producing a coating film, preferably a method for producing a coating film compatible with biomaterials (for example, having an adhesion suppressing ability to biomaterials and a cell culture promoting property).

### BACKGROUND ART

It has been proposed coating materials having an ability to suppress adhesion of various biological substances to suppress adhesion of biological substances to medical instruments, equipment, etc., such as an artificial dialyzer, artificial organs and medical instruments, etc., or coating materials for promoting cell culture.

In Patent Document 1, ion complex materials having an ability to control adhesion of biological substances and a method for producing the same have been disclosed. In Patent Document 2, surface treatment agents excellent in characteristics such as antifouling property, etc., have been disclosed.

### PRIOR ART DOCUMENTS

### PATENT DOCUMENTS

Patent Document 1: WO 2014/196650
Patent Document 2: WO 2018/008663

### SUMMARY OF THE INVENTION

### PROBLEMS TO BE SOLVED BY THE INVENTION

The phosphoric acid ester monomer having an unsaturated bond(s) contains a large amount of impurities such as phosphodiester, etc., according to a usual synthetic method. When a phosphoric acid ester monomer having an unsaturated bond(s), which contains a large amount of a phosphodiester component, and another different monomer having an unsaturated bond are used for copolymerization reaction, there is a problem that a three-dimensional network structure in which the phosphodiester becomes a crosslinked portion is formed and gelation occurs so that it cannot be made into a varnish in which the polymer is dissolved or dispersed in a solvent.

The inventors of the present application have earnestly studied and as a result, they have found that a copolymer-containing varnish can be produced by copolymerizing a phosphoric acid ester monomer having a specific structure and a certain purity or higher and another different monomer having an unsaturated bond by a known method per se without gelation.

The present invention is to provide a method for producing a copolymer-containing varnish which does not cause the problem of gelation, a method for producing a composition for forming a coating film, a method for producing a coating film, preferably a method for producing a coating film compatible with biomaterials (for example, having an adhesion suppressing ability to biomaterials and a cell culture promoting property).

### MEANS TO SOLVE THE PROBLEMS

The present invention includes the following.
[1] A method for producing a varnish containing a copolymer which comprises a step of copolymerizing a monomer mixture containing compounds represented by the following formula (1) and the formula (2): 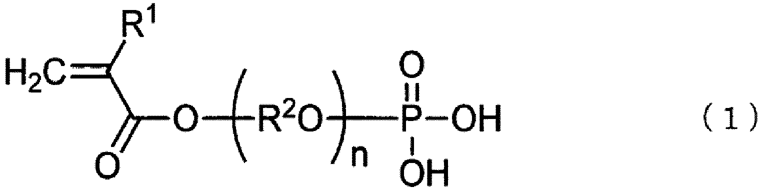 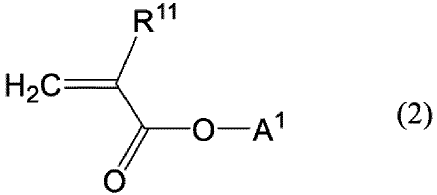 (in the formula (1), R¹ represents a hydrogen atom or a methyl group, R² represents an alkylene group having 1 to 6 carbon atoms, n represents an integer of 1 to 30, in the formula (2), R¹¹ represents a hydrogen atom or a methyl group, and A¹ represents a monovalent organic group having a cationic property) in a solvent for polymerization, in the presence of a radical polymerization initiator,
   wherein % by mass of the compound represented by the formula (1) in a phosphorus-containing compound contained in the above-mentioned monomer mixture is 70% by mass or more.
[2] The method for producing a varnish containing a copolymer described in [1], wherein the above-mentioned A¹ contains the structure represented by the following formula (2-1): 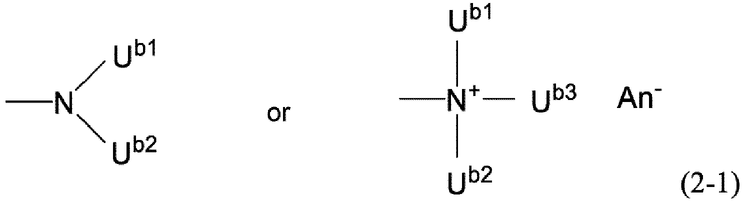 (U^{b1}, U^{b2} and U^{b3} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion).
[3] The method for producing a varnish containing a copolymer described in [1], wherein the above-mentioned A¹ is represented by the following formula (2-2): 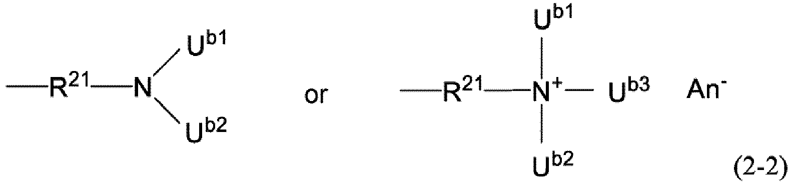 (R²¹ represents an alkylene group having 1 to 6 carbon atoms which may be interrupted by a phosphodiester bond, U^{b1}, U^{b2} and U^{b3} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion).
[4] A method for producing a composition for forming a coating film which comprises a step of copolymerizing a monomer mixture containing compounds represented by the following formula (1) and the formula (2): 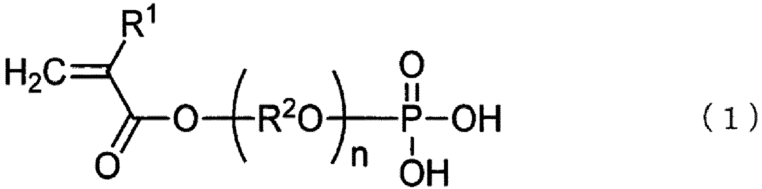 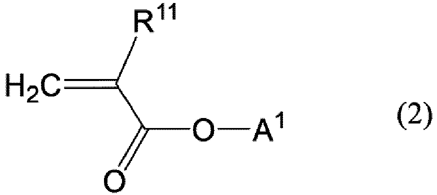 (in the formula (1), R¹ represents a hydrogen atom or a methyl group, R² represents an alkylene group having 1 to 6 carbon atoms, n represents an integer of 1 to 30, in the formula (2), R¹¹ represents a hydrogen atom or a methyl group, and A¹ represents a monovalent organic group having a cationic property.) in a solvent for polymerization, in the presence of a radical polymerization initiator, and then, a step of adding a solvent,
   wherein % by mass of the compound represented by the formula (1) in a phosphorus-containing compound contained in the above-mentioned monomer mixture is 70% by mass or more.
[5] The method for producing a composition for forming a coating film described in [4], which is compatible with biomaterials.
[6] A method for producing a coating film, which comprises a step of coating the composition for forming a coating film described in [4] or [5] to a substrate.

### EFFECTS OF THE INVENTION

According to the method of the present invention, it can be provided a method for producing a copolymer-containing varnish which does not cause the problem of gelation, a method for producing a composition for forming a coating film, a method for producing a coating film, preferably a method for producing a coating film compatible with biomaterials (for example, having an adhesion suppressing ability to biomaterials and a cell culture promoting property).

### BRIEF DESCRIPTION OF THE DRAWINGS

FIG. 1 is a photomicrograph showing a cell adhesion inhibitory effect of a well coated with the composition for forming a coating film of Example 1 in Test Example 1.
FIG. 2 is a photomicrograph showing a cell adhesion inhibitory effect of a well coated with the composition for forming a coating film of Example 2 in Test Example 1.
FIG. 3 is a photomicrograph showing a cell adhesion inhibitory effect of a well coated with the composition for forming a coating film of Example 3 in Test Example 1.
FIG. 4 is a photomicrograph showing a cell adhesion inhibitory effect of a well coated with the composition for forming a coating film of a positive control (Reference Example 1) in Test Example 1.
FIG. 5 is a photomicrograph showing a cell adhesion inhibitory effect of a well of a negative control (uncoated) in Test Example 1.

### EMBODIMENTS TO CARRY OUT THE INVENTION

### «Explanation of the terms»

The terms used in the present invention have the following definitions, otherwise specifically mentioned.

The terms "alkylene group having 1 to 6 carbon atoms" may be mentioned a methylene group, an ethylene group, an n-propylene (trimethylene) group, a propylene group, a cyclopropylene group, an n-butylene group, an isobutylene group, an s-butylene group, a t-butylene group, a cyclobutylene group, a 1-methyl-cyclopropylene group, a 2-methyl-cyclopropylene group, an n-pentylene group, a 1-methyl-n-butylene group, a 2-methyl-n-butylene group, a 3-methyl-n-butylene group, a 1,1-dimethyl-n-propylene group, a 1,2-dimethyl-n-propylene group, a 2,2-dimethyl-n-propylene group, a 1-ethyl-n-propylene group, a cyclopentylene group, a 1-methyl-cyclobutylene group, a 2-methyl-cyclobutylene group, a 3-methyl-cyclobutylene group, a 1,2-dimethyl-cyclopropylene group, a 2,3-dimethyl-cyclopropylene group, a 1-ethyl-cyclopropylene group, a 2-ethyl-cyclopropylene group, an n-hexylene group, a 1-methyl-n-pentylene group, a 2-methyl-n-pentylene group, a 3-methyl-n-pentylene group, a 4-methyl-n-pentylene group, a 1,1-dimethyl-n-butylene group, a 1,2-dimethyl-n-butylene group, a 1,3-dimethyl-n-butylene group, a 2,2-dimethyl-n-butylene group, a 2,3-dimethyl-n-butylene group, a 3,3-dimethyl-n-butylene group, a 1-ethyl-n-butylene group, a 2-ethyl-n-butylene group, a 1,1,2-trimethyl-n-propylene group, a 1,2,2-trimethyl-n-propylene group, a 1-ethyl-1-methyl-n-propylene group, a 1-ethyl-2-methyl-n-propylene group, a cyclohexylene group, a 1-methyl-cyclopentylene group, a 2-methyl-cyclopentylene group, a 3-methyl-cyclopentylene group, a 1-ethyl-cyclobutylene group, a 2-ethyl-cyclobutylene group, a 3-ethyl-cyclobutylene group, a 1,2-dimethyl-cyclobutylene group, a 1,3-dimethyl-cyclobutylene group, a 2,2-dimethyl-cyclobutylene group, a 2,3-dimethyl-cyclobutylene group, a 2,4-dimethyl-cyclobutylene group, a 3,3-dimethyl-cyclobutylene group, a 1-n-propyl-cyclopropylene group, a 2-n-propyl-cyclopropylene group, a 1-isopropyl-cyclopropylene group, a 2-isopropyl-cyclopropylene group, a 1,2,2-trimethyl-cyclopropylene group, a 1,2,3-trimethyl-cyclopropylene group, a 2,2,3-trimethyl-cyclopropylene group, a 1-ethyl-2-methyl-cyclopropylene group, a 2-ethyl-1-methyl-cyclopropylene group, a 2-ethyl-2-methyl-cyclopropylene group and a 2-ethyl-3-methyl-cyclopropylene group, etc.

Also, the terms "alkylene group having 1 to 6 carbon atoms which may be interrupted by a phosphodiester bond" mean an "alkylene group having 1 to 6 carbon atoms" or an "alkylene group having 1 to 6 carbon atoms interrupted by a phosphodiester bond".

Examples of the "alkylene group having 1 to 6 carbon atoms" are as mentioned above. The "alkylene group having 1 to 6 carbon atoms interrupted by a phosphodiester bond" mean a divalent group in which at least one methylene (-CH₂-) group of the above-mentioned "alkylene group having 1 to 6 carbon atoms" is replaced with a phosphodiester bond.

The "linear or branched alkyl group having 1 to 5 carbon atoms" may be mentioned a methyl group, an ethyl group, an n-propyl group, an isopropyl group, an n-butyl group, an isobutyl group, an s-butyl group, a t-butyl group, an n-pentyl group, a 1-methylbutyl group, a 2-methylbutyl group, a 3-methylbutyl group, a 1,1-dimethylpropyl group, a 1,2-dimethylpropyl group, a 2,2-dimethylpropyl group or a 1-ethylpropyl group.

The "halide ion" may be mentioned a fluoride ion, a chloride ion, a bromide ion or an iodide ion.

The "inorganic acid ion" may be mentioned a carbonate ion, a sulfate ion, a phosphate ion, a hydrogen phosphate ion, a dihydrogen phosphate ion, a nitrate ion, a perchlorate ion or a borate ion.

The "solvent for polymerization" is a solvent used for copolymerization is a solvent used for a copolymerization reaction known *per se* of two or more kinds of monomers of the present invention, and may be water, a phosphate buffer or an alcohol such as ethanol, etc., or a mixed solvent in which these are combined, and it is desirable to contain water or ethanol. Further, it is preferable to contain 10% by mass or more and 100% by mass or less of water or ethanol. Moreover, it is preferable to contain 50% by mass or more and 100% by mass or less of water or ethanol. Furthermore, it is preferable to contain 80% by mass or more and 100% by mass or less of water or ethanol. Still further, it is preferable to contain 90% by mass or more and 100% by mass or less of water or ethanol. It is preferable that the sum of water and ethanol is 100% by mass.

The "radical polymerization initiator" may be a "heat radical polymerization initiator" or a "photoradical polymerization initiator".

As the "heat radical polymerization initiator", 2,2'-azobis(isobutyronitrile), 2,2'-azobis(2-methylbutyronitrile), 2,2'-azobis(2,4-dimethylvaleronitrile) (product name of FUJIFILM Wako Pure Chemical Corporation; V-65, 10 hour half-life temperature; 51°C), 4,4'-azobis(4-cyanovaleric acid), 2,2'-azobis(4-methoxy-2,4-dimethylvaleronitrile), 1,1'-azobis(cyclohexane-1-carbonitrile), 1-[(1-cyano-1-methylethyl)azo]formamide, 2,2'-azobis[2-(2-imidazolin-2-yl)propane] dihydrochloride (FUJIFILM Wako Pure Chemical Corporation; VA-044, 10 hour half-life temperature; 44°C), 2,2'-azobis[2-(2-imidazolin-2-yl)propane] (FUJIFILM Wako Pure Chemical Corporation; VA-061, 10 hour half-life temperature; 61°C), 2,2'-azobis(2-methylpropionamidine) dihydrochloride, 2,2'-azo(2-methyl-N-(2-hydroxyethyl)propionamide (product name of FUJIFILM Wako Pure Chemical Corporation; VA-086, 10 hour half-life temperature; 86°C), benzoyl peroxide (BPO), 2,2'-azobis(N-(2-carboxyethyl)-2-methylpropionamidine) n-hydrate (product name of FUJIFILM Wako Pure Chemical Corporation; VA-057, 10 hour half-life temperature; 57°C), 4,4'-azobis(4-cyanopentanoic acid) (product name of FUJIFILM Wako Pure Chemical Corporation; VA-501), 2,2'-azobis[2-(2-imidazolin-2-yl)propane]disulfate dihydrate (product name of FUJIFILM Wako Pure Chemical Corporation; VA-046B, 10 hour half-life temperature; 46°C), 2,2'-azobis(2-amidinopropane) dihydrochloride (product name of FUJIFILM Wako Pure Chemical Corporation; V-50, 10 hour half-life temperature; 56°C), peroxydisulfuric acid or t-butyl hydroperoxide, dimethyl 1,1'-azobis(1-cyclohexanecarboxylate) (product name of FUJIFILM Wako Pure Chemical Corporation; VE-073), etc., are used.

As the "photoradical polymerization initiator", acetophenones such as acetophenone, chloroacetophenone, hydroxyacetophenone, 2-aminoacetophenone, dialkylaminoacetophenone, 2,2-diethoxyacetophenone, 2,2-dimethoxy-2'-phenylacetophenone (product name of BASF; Irgacure 651), 2-hydroxy-2-methyl-1-phenylpropanone (product name of BASF; Irgacure 1173), 1-[4-(2-hydroxyethoxy)-phenyl]-2-hydroxymethylpropanone (product name of BASF; Irgacure 2959), 2-hydroxy-1-{4-[4-(2-hydroxy-2-methylpropionyl)benzyl]phenyl}-2-methyl-1-propan-1-one (product name of BASF; Irgacure 127), 2-methyl-1-[4-(methylthio)phenyl]-2-morpholinopropan-1-one (product name of BASF; Irgacure 907), 2-benzyl-2-(dimethylamino)-4-morpholinobutyrophenone (product name of BASF; Irgacure 369), etc.; benzoins such as benzoin, benzoin methyl ether, benzoin ethyl ether, benzoin isopropyl ether, benzoin isobutyl ether, 1-hydroxycyclohexyl phenyl ketone (product name of BASF; Irgacure 184), etc.; benzophenones such as benzophenone, benzoylbenzoic acid, methyl benzoylbenzoate, methyl-o-benzoylbenzoate, 4-phenylbenzophenone, hydroxybenzophenone, hydroxypropylbenzophenone, acryl benzophenone, 4,4'-bis(dimethylamino)benzophenone, 4,4'-dichlorobenzophenone, etc.; thioxanthones such as thioxanthone, 2-chlorothioxanthone, 2-methylthioxanthone, diethylthioxanthone, dimethylthioxanthone, etc.; 2,4,6-trimethylbenzoyl-diphenylphosphine oxide (product name of BASF; Irgacure TPO), bis(2,4,6-trimethylbenzoyl)-phenylphosphine oxide (product name of BASF; Irgacure 819), etc., acylphosphine oxides; a mixture of oxyphenylacetic acid 2-[2-oxo-2-phenylacetoxyethoxy]ethyl ester and oxyphenylacetic acid 2-(2-hydroxyethoxy)ethyl ester (product name of BASF; Irgacure 754), methyl benzoylformate (product name of BASF; Irgacure MBF), α-acyloxime ester, benzyl-(o-ethoxycarbonyl)-α-monoxime, glyoxylate, 2-ethylanthraquinone, camphorquinone, tetramethylthiuram sulfide, azobisisobutyronitrile, benzoyl peroxide, dialkyl peroxide, tert-butyl peroxypivalate, etc., are mentioned.

The "solvent" is a solvent used for the composition for forming a coating film of the present invention, and may be mentioned water, phosphate buffered physiological saline (PBS), and an alcohol. As the alcohol, there may be mentioned an alcohol having 2 to 6 carbon atoms, for example, ethanol, propanol, isopropanol, 1-butanol, 2-butanol, isobutanol, t-butanol, 1-pentanol, 2-pentanol, 3-pentanol, 1-heptanol, 2-heptanol, 2,2-dimethyl-1-propanol (=neopentyl alcohol), 2-methyl-1-propanol, 2-methyl-1-butanol, 2-methyl-2-butanol (=t-amyl alcohol), 3-methyl-1-butanol, 3-methyl-3-pentanol, cyclopentanol, 1-hexanol, 2-hexanol, 3-hexanol, 2,3-dimethyl-2-butanol, 3,3-dimethyl-1-butanol, 3,3-dimethyl-2-butanol, 2-ethyl-1-butanol, 2-methyl-1-pentanol, 2-methyl-2-pentanol, 2-methyl-3-pentanol, 3-methyl-1-pentanol, 3-methyl-2-pentanol, 3-methyl-3-pentanol, 4-methyl-1-pentanol, 4-methyl-2-pentanol, 4-methyl-3-pentanol and cyclohexanol, which may be used singly or a mixed solvent of a combination of these, and from the viewpoint of solubility of the copolymer, it is preferably selected from water, PBS, ethanol and propanol.

The terms "biological substance" may be mentioned a protein, a saccharide, a nucleic acid, a cell or a combination thereof. For example, the protein may be mentioned fibrinogen, bovine serum albumin (BSA), human albumin, various kinds of globulins, β-lipoprotein, various kinds of antibodies (IgG, IgA and IgM), peroxidase, various kinds of complements, various kinds of lectins, fibronectin, lysozyme, von Willebrand factor (vWF), serum γ-globulin, pepsin, ovalbumin, insulin, histone, ribonuclease, collagen and cytochrome c, the saccharide may be mentioned, for example, glucose, galactose, mannose, fructose, heparin and hyaluronic acid, the nucleic acid may be mentioned, for example, deoxyribonucleic acid (DNA) and ribonucleic acid (RNA), the cell may be mentioned, for example, fibroblast, bone marrow cells, B lymphocytes, T lymphocytes, neutrophils, red blood cells, platelets, macrophages, monocytes, bone cells, pericytes, dendritic cells, keratinocytes, fat cells, mesenchymal cells, epithelial cells, epidermal cells, endothelial cells, vascular endothelial cells, hepatic parenchymal cells, cartilage cells, cumulus cells, neural cells, glial cells, neurons, oligodendrocytes, microglia, astroglial cells, heart cells, esophagus cells, muscle cells (for example, smooth muscle cells or skeletal muscle cells), pancreatic beta cells, melanocytes, hematopoietic precursor cells, mononuclear cells, embryonic stem cells (ES cell), embryonic tumor cells, embryonic germline stem cells, induced pluripotent stem cells (iPS cell), neural stem cells, hematopoietic stem cells, mesenchymal stem cells, liver stem cells, pancreatic stem cells, muscle stem cells, germline stem cells, intestinal stem cells, cancer stem cells, hair follicle stem cells and various kinds of cell lines (for example, HCT116, Huh7, HEK293 (human embryonic kidney cell), HeLa (human cervical cancer cell lines), HepG2 (human liver cancer cell lines), UT7/TPO (human leukemia cell lines), CHO (Chinese hamster ovary cell lines), MDCK, MDBK, BHK, C-33A, HT-29, AE-1, 3D9, Ns0/1, Jurkat, NIH3T3, PC12, S2, Sf9, Sf21, High Five and Vero), etc.

The terms "having an ability to inhibit adhesion of the biological substance" mean that, for example,
when the biological substance is a platelet, it means that a relative platelet adhered number (%) ((platelet adhered number (number) of Example)/(platelet adhered number (number) of Comparative Example)) when compared with no coating film by the platelet adhesion test carried out by the method described in Example of WO 2016/093293A1 is 50% or less, preferably 30% or less, and further preferably 20% or less;
when the biological substance is a protein, it means that a mass (%) per a relative unit area ((a mass per a unit area of Example (ng/cm²)/(a mass per a unit area of Comparative Example (ng/cm²))) when compared with no coating film by the QCM-D measurement carried out by the method described in Example is 50% or less, preferably 30% or less, and further preferably 20% or less; or
when the biological substance is a cell, it means that a relative absorbance (WST O.D. 450 nm) (%) ((absorbance of Example (WST O.D. 450 nm))/(absorbance of Comparative Example (WST O.D. 450 nm))) when compared with no coating film by the fluorescence microscope carried out by the method described in Example of WO 2016/093293A1 is 50% or less, preferably 30% or less, and further preferably 20% or less.

### <Method for producing copolymer-containing varnish>

The present invention is directed to a method for producing a copolymer-containing varnish which comprises a step of copolymerizing a monomer mixture containing compounds represented by the following formula (1) and the formula (2): 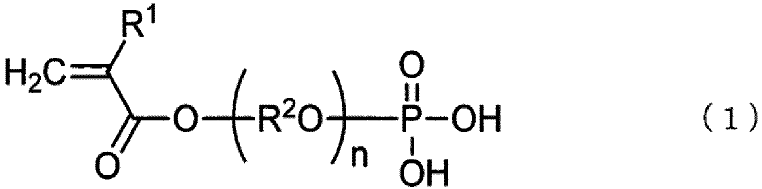 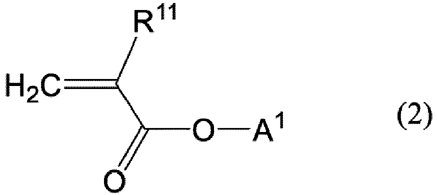 (in the formula (1), R¹ represents a hydrogen atom or a methyl group, R² represents an alkylene group having 1 to 6 carbon atoms, n represents an integer of 1 to 30, in the formula (2), R¹¹ represents a hydrogen atom or a methyl group, and A¹ represents a monovalent organic group having a cationic property) in a solvent for polymerization in the presence of a radical polymerization initiator,
wherein % by mass (purity) of the compound represented by the formula (1) in a phosphorus-containing compound contained in the above-mentioned monomer mixture is 70% by mass or more.

In the present invention, the terms "monomer mixture" refer to a mixture containing all the compounds applied to the copolymerization reaction step in addition to all the compounds represented by the above-mentioned formula (1) and formula (2). That is, the "% by mass (absolute % by mass) of the compound represented by the formula (1) in a phosphorus-containing compound contained in the monomer mixture" can be said, in other words, that the purity (content) of the compound represented by the formula (1) based on the total amount of the phosphorus-containing compound contained in the monomer mixture applied to the copolymerization reaction step is 70% by mass or more.

The above-mentioned phosphorus-containing compound means a compound containing at least one phosphorus atom in one molecule of the compound, and in addition to the compound represented by the formula (1), it is impurities (for example, phosphodiester compound corresponding to a dimer of the compound represented by the formula (1), etc.) derived from the compound represented by the formula (1).
n represents an integer of 1 to 30, preferably an integer of 1 to 20, an integer of 1 to 10, an integer of 1 to 7, an integer of 2 to 6, and an integer of 3 to 5.

As the compound represented by the formula (1), a monomer described in JP 2018-27927A may be used.

The % by mass (absolute % by mass) of the compound represented by the formula (1) in the phosphorus-containing compound containing the monomer mixture used for producing the above-mentioned copolymer can be obtained, for example, by compositional analysis of the phosphorus-containing compound described in Examples.

The % by mass (absolute % by mass) of the compound represented by the formula (1) in the phosphorus-containing compound is preferably 70% by mass or more, 75% by mass or more, 80% by mass or more, 85% by mass or more, 90% by mass or more and 95% by mass or more. It is most preferably 100% by mass.

A ratio of the repeating unit derived from the compound represented by the formula (1) in the copolymer according to the present invention is, when R²¹ of the compound represented by the formula (2) mentioned later is an alkylene group having 1 to 6 carbon atoms which is interrupted by a phosphodiester bond, 5 mol% to 80 mol%, preferably 7 mol% to 70 mol%, preferably 8 mol% to 65 mol%, and further preferably 10 mol% to 60 mol%.

A ratio of the repeating unit derived from the compound represented by the formula (1) in the copolymer according to the present invention is, when R²¹ of the compound represented by the formula (2) mentioned later is not interrupted by a phosphodiester bond (that is, R²¹ is an alkylene group having 1 to 6 carbon atoms), 10 mol% to 80 mol%, preferably 30 mol% to 70 mol%, and further preferably 40 mol% to 60 mol%.

Incidentally, the copolymer according to the present invention may contain two or more kinds of repeating units derived from the compound represented by the formula (1), preferably 1 kind or 2 kind, and preferably 1 kind.

A ratio of the repeating unit derived from the compound represented by the formula (2) in the copolymer according to the present invention may be the entire balance obtained by subtracting the above-mentioned formula (1) from the whole copolymer, or may be the balance obtained by subtracting the total ratio of the above-mentioned formula (1) and the third component mentioned below. Incidentally, the copolymer according to the present invention may contain two or more kinds of the repeating units derived from the compounds represented by the formula (2).

In the monomer mixture according to the present invention, as a further optional third component, an ethylenically unsaturated monomer, or a polysaccharide or derivatives thereof may be further contained. Examples of the ethylenically unsaturated monomer may be mentioned one kind or two or more kinds of the ethylenically unsaturated monomers selected from the group consisting of a (meth)acrylic acid and an ester thereof; vinyl acetate; vinylpyrrolidone; ethylene; vinyl alcohol; and a hydrophilic functional derivative thereof. Examples of the polysaccharides or derivatives thereof may be mentioned a cellulose polymer such as hydroxyalkyl cellulose (for example, hydroxy ethyl cellulose or hydroxypropyl cellulose), etc., starch, dextran and curdlan.

The hydrophilic functional derivative refers to an ethylenically unsaturated monomer having a hydrophilic functional group or structure. Examples of the hydrophilic functional group or structure may be mentioned an amide structure; an alkylene glycol residue; an amino group; and a sulfinyl group, etc.

The amide structure means a group represented by the following formula: 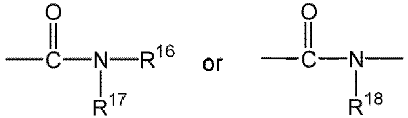 [here, R¹⁶, R¹⁷ and R¹⁸ each independently are a hydrogen atom or an organic group (for example, a methyl group, a hydroxymethyl group or a hydroxyethyl group, etc.)]. Examples of the ethylenically unsaturated monomer having such a structure may be mentioned (meth)acrylamide, N-(hydroxymethyl) (meth)acrylamide, etc. Further, the monomer or polymer having such a structure is disclosed in, for example, JP 2010-169604A, etc.

The alkylene glycol residue means an alkyleneoxy group (-Alk-O-) remained after condensation reaction of a hydroxy group(s) at one end terminal or both ends of alkylene glycol (HO-Alk-OH; here, Alk is an alkylene group having 1 to 10 carbon atoms) with another compound, and a poly(alkyleneoxy) group in which alkyleneoxy units are repeated is also included. Examples of the ethylenically unsaturated monomer having such a structure may be mentioned 2-hydroxyethyl(meth)acrylate, methoxypolyethylene glycol (meth)acrylate, etc. Further, the monomer or polymer having such a structure is disclosed in, for example, JP 2008-533489A.

The amino group means a group represented by the formula: -NH₂, -NHR¹⁹ or -NR²⁰R²¹ [here, R¹⁹, R²⁰ and R²¹ are each independently an organic group (for example, a linear or branched alkyl group having 1 to 5 carbon atoms, etc.)]. In the amino group of the present invention, quaternized or chlorinated amino group is included. Examples of the ethylenically unsaturated monomer having such a structure may be mentioned dimethylaminoethyl (meth)acrylate, 2-(t-butylamino)ethyl (meth)acrylate, methacryloylcholine chloride, etc.

The sulfinyl group means a group represented by the following formula: 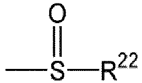 [here, R²² is an organic group (for example, an organic group having 1 to 10 carbon atoms, preferably an alkyl group having 1 to 10 carbon atoms which has one or more hydroxy groups, etc.)].
As a polymer having such a structure, there may be mentioned a copolymer disclosed in JP 2014-48278A, etc.

### (Weight average molecular weight of copolymer)

The weight molecular weight of the copolymer according to the present invention may be several thousands to several millions or so, and preferably 5,000 to 5,000,000. It is further preferably 10,000 to 2,000,000. In addition, it may be either of a random copolymer, a block copolymer or a graft copolymer, there is no particular limitation on the copolymerization reaction itself for producing the copolymer, and a conventionally known method synthesizing in a solution can be used such as polymerization utilizing radical polymerization, ionic polymerization, photopolymerization, macromer, emulsion polymerization, etc. These can be used any of the copolymers of the present invention solely or a plurality of the copolymers are mixed and the ratio thereof may be changed depending on the objective uses.

### (Copolymerization reaction)

The copolymer-containing varnish of the present invention can be produced by a producing method which contains a step of reacting (polymerizing) the monomer mixture containing the compounds represented by the above-mentioned formulae (1) and (2) in a solvent for polymerization and a total concentration of the both compounds of 0.01% by mass to 20% by mass.

An amount of the radical polymerization initiator to be added is 0. 05% by mass to 10% by mass based on the total weight of the monomers used for polymerization.

As the reaction conditions, the copolymer of the present invention can be obtained by heating a reaction vessel by an oil bath, etc., to 50°C to 200°C and subjecting to stirring for 1 hour to 48 hours, more preferably at 80°C to 150°C for 5 hours to 30 hours to proceed the polymerization reaction. A reaction atmosphere may be under atmospheric condition, and a nitrogen atmosphere is preferable.

As the reaction procedure, the compounds represented by the formula (1) and the formula (2) and, if necessary, a third component monomer(s) may be all charged into a solvent for polymerization at room temperature, and polymerized by heating to the above-mentioned temperature, or all or a part of the mixture of the compounds represented by the formula (1) and the formula (2) and, if necessary, a third component monomer(s) may be added dropwise to a preheated solvent for polymerization little by little.

According to the latter reaction procedure, the copolymer-containing varnish of the present invention can be prepared by a producing method which comprises steps of adding dropwise a mixture containing a monomer mixture which contains the compounds represented by the above-mentioned formula (1) and formula (2), a solvent for polymerization and a polymerization initiator to a solvent which is retained at a temperature higher than the 10 hour half-life temperature of the polymerization initiator, and reacting (polymerizing) the same.

In the above-mentioned A¹, the monovalent organic group having cationic property is an organic group having cationic property, and for example, there may be mentioned a group containing tertiary amine and quaternary ammonium salt. Also, the above-mentioned A¹ may be a cationic group containing an aromatic ring, and for example, there may be mentioned a group containing a pyridine skeleton, etc.

The above-mentioned A¹ preferably contains a structure represented by the following formula (2-1): 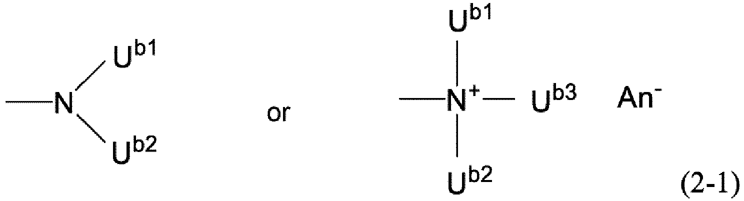 (U^{b1}, U^{b2} and U^{b3} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion).

As the above-mentioned An⁻, preferred are a halide ion, a sulfate ion, a phosphate ion, a hydroxide ion and an isothiocyanate ion, and particularly preferred is a halide ion.

The above-mentioned A¹ is preferably represented by the following formula (2-2): 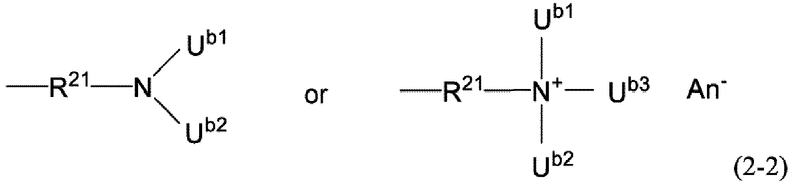 (R²¹ represents an alkylene group having 1 to 6 carbon atoms which may be interrupted by a phosphodiester bond, U^{b1}, U^{b2} and U^{b3} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion).

The structure represented by the above-mentioned formula (2-2) may be a betaine structure. The betaine structure means a monovalent or divalent group of a compound having an amphoteric center of a quaternary ammonium type cationic structure and an acidic anionic structure, and there may be mentioned, for example, a phosphorylcholine group: 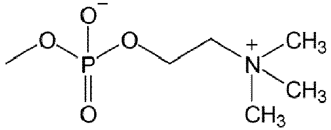 Examples of the compound represented by the formula (2) having such a structure may be mentioned 2-methacryloyloxyethylphosphorylcholine (MPC), etc.

### <Method for producing composition for forming coating film>

It is a method for producing a composition for forming a coating film, which comprises a step of copolymerizing a monomer mixture containing compounds represented by the following formula (1) and the formula (2): 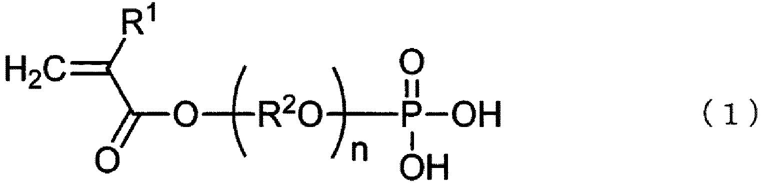 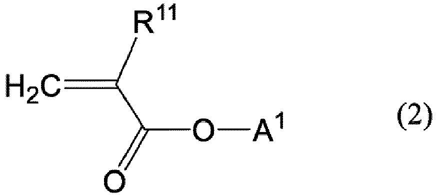 (in the formula (1), R¹ represents a hydrogen atom or a methyl group, R² represents an alkylene group having 1 to 6 carbon atoms, n represents an integer of 1 to 30, in the formula (2), R¹¹ represents a hydrogen atom or a methyl group, and A¹ represents a monovalent organic group having a cationic property) in a solvent for polymerization in the presence of a radical polymerization initiator, and then, a step of adding a solvent,
wherein % by mass (purity) of the compound represented by the formula (1) in the phosphorus-containing compound contained in the above-mentioned monomer mixture is 70% by mass or more. According to the method of the present invention, it is possible to produce a composition for forming a coating film in which the copolymer is stably dissolved or uniformly dispersed in a solvent without causing gelation of the copolymer, etc.

The composition for forming a coating film of the present invention is produced by copolymerizing a monomer mixture containing each compound as mentioned above, and then, adding the above-mentioned solvent by a method known *per se.*

The method for producing the composition for forming a coating film of the present invention is preferably a method for producing a composition for forming a coating film compatible with biomaterials.

The terms "compatible with biomaterials" mean that it has, in addition to the above-mentioned ability to suppress adhesion of the biological substances, cell culture promoting properties (for example, a polymer for coating a cell culture container as described in JP 2014-162865A, and a material for forming a base film for cell culture as described in Japanese Patent Application No. 2018-157444).

A concentration of the solid content in the composition for forming a coating film according to the present invention is desirably 0. 01 to 50% by mass to form the coating film uniformly. In addition, a concentration of the copolymer in the composition for forming a coating film is preferably 0. 01 to 4% by mass, more preferably 0. 01 to 3% by mass, particularly preferably 0. 01 to 2% by mass, and further preferably 0. 01 to 1% by mass. If the concentration of the copolymer is 0. 01% by mass or less, the concentration of the copolymer of the resulting composition for forming a coating film is too low and a coating film having a sufficient film thickness cannot be formed, while if it is 4% by mass or more, storage stability of the composition for forming a coating film becomes poor, and there is a possibility of causing precipitation of the dissolved materials or gelation.

In order to adjust ion balance of the copolymer in the composition for forming a coating film according to the present invention, at the time of obtaining a coating film of the present invention, a step of adjusting a pH in the composition for forming a coating film in advance may be further contained. Adjustment of the pH may be carried out, for example, by adding a pH adjusting agent to the composition containing the above-mentioned copolymer and solvent, and made the pH of the composition 3.0 to 13.5, preferably 3.5 to 8.5, and further preferably 3.5 to 7.0, or may be carried out by making it preferably 8.5 to 13.5, and further preferably 10.0 to 13.5. A kind of the usable pH adjusting agent and an amount thereof can be optionally selected depending on the concentration of the above-mentioned copolymer or an existing ratio of the anion and the cation thereof, and the like.

Examples of the pH adjusting agent may be mentioned organic amines such as ammonia, diethanolamine, pyridine, N-methyl-D-glucamine, tris(hydroxymethyl)-aminomethane, etc.; alkali metal hydroxides such as potassium hydroxide, sodium hydroxide, etc.; alkali metal halides such as potassium chloride, sodium chloride, etc.; inorganic acids such as sulfuric acid, phosphoric acid, hydrochloric acid, carbonic acid, etc., or alkali metal salts thereof; quaternary ammonium cations such as choline, etc.; or a mixture thereof (for example, a buffer such as phosphate buffered saline, etc.). Among these, ammonia, diethanolamine, sodium hydroxide, choline, N-methyl-D-glucamine, tris(hydroxymethyl)aminomethane are preferable, and in particular, ammonia, diethanolamine, sodium hydroxide and choline are preferable.

Accordingly, the present invention relates to a method for producing a composition for forming a coating film which comprises (i) a copolymer containing a recurring unit derived from the compound represented by the above-mentioned formula (1) and a recurring unit derived from the compound represented by the above-mentioned formula (2), (ii) a solvent, and, if necessary, and (iii) a pH adjusting agent. Specific examples of the copolymer, solvent and pH adjusting agent are as mentioned above.

Further, other substances may be added to the composition for forming a coating film of the present invention in addition to the above-mentioned copolymer and the solvent within the range that does not impair the properties of the obtainable coating film, if necessary. As the other substances, there may be mentioned preservatives, surfactants, primers that enhance adhesive property with the substrate, fungicides and sugars, etc.

### <Method for producing coating film>

The method for producing a coating film of the present invention is a method for producing a coating film which comprises a step of coating the above-mentioned composition for forming a coating film on a substrate.

The composition for forming a coating film according to the present invention is coated on the substrate, and dried to form a coating film.

As the substrate for forming the coating film of the present invention, there may be mentioned glass, a metal containing compound or a semi-metal containing compound, activated carbon or a resin. The metal containing compound or the semi-metal containing compound may be mentioned, for example, ceramics comprising a metal oxide as a basic component, which are a sintered body baked by a heat treatment at a high temperature, a semiconductor such as silicon, an inorganic solid material including a molded product of an inorganic compound such as a metal oxide or a semi-metal oxide (silicon oxide, alumina, etc.), a metal carbide or a semi-metal carbide, a metal nitride or a semi-metal nitride (silicon nitride, etc.), a metal boride or a semi-metal boride, etc., aluminum, nickel-titanium and stainless (SUS304, SUS316, SUS316L, etc.).

As the resin, it may be either of a natural resin or a synthetic resin, as the natural resin, there may be mentioned cellulose, cellulose triacetate (CTA), cellulose to which dextran sulfate has been fixed, etc., and as the synthetic resin, there may be preferably used polyacrylonitrile (PAN), polyester-based polymer alloy (PEPA), polystyrene (PS), polysulfone (PSF), polyethylene terephthalate (PET), polymethyl methacrylate (PMMA), polyvinyl alcohol (PVA), polyurethane (PU), ethylene vinyl alcohol (EVAL), polyethylene (PE), polyester (PE), polypropylene (PP), polyvinylidene fluoride (PVDF), polyether sulfone (PES), polycarbonate (PC), polyvinyl chloride (PVC), polytetrafluoroethylene (PTFE), ultra-high molecular weight polyethylene (UHPE), polydimethylsiloxane (PDMS), acrylonitrile-butadienestyrene resin (ABS), Teflon (Registered Trademark), cycloolefin polymer (COP) (for example, ZEONOR (Registered Trademark), ZEONEX (Registered Trademark) (available from ZEON CORPORATION)) or various kinds of ion-exchange resins, etc., more preferably polystyrene (PS), polyether sulfone (PES), polypropylene (PP) and cycloolefin polymer (COP), and particularly preferably polystyrene (PS) and polyether sulfone (PES). The coating film of the present invention can be formed by low temperature drying, so that it can be applied to a resin having low heat resistance, etc.

In order to form the coating film of the present invention, the above-mentioned composition for forming a coating film is coated on at least a part of the surface of the substrate. A coating method is not particularly limited, and a usual coating method such as spin coating, dip coating, solvent casting method, etc., is used.

The drying step of the coating film according to the present invention is carried out under atmosphere or under vacuum, preferably at a temperature in the range of
-200°C to 200°C. By the drying step, the solvent in the above-mentioned composition for forming a coating film is removed, and the phosphoric acid portion of the compound represented by the formula (1) and the cationic portion of the compound represented by the formula (2) of the copolymer according to the present invention form an ionic bond to completely fixed to the substrate.

The coating film can be formed, for example, by drying at room temperature (10°C to 35°C, for example, 25°C), but in order to form the coating film more quickly, it may be dried, for example, at 40°C to 50°C. In addition, it may be employed a drying step at an extremely low temperature to a low temperature (around -200°C to around
-30°C) by the freeze-drying method. Freeze-drying is called as vacuum freeze-drying, and is a method in which a material desired to be dried is usually cooled with a refrigerant and the solvent is removed by sublimation in a vacuum state. The common refrigerants used in freeze-drying may be mentioned a mixed medium of dry ice and methanol (-78°C), liquid nitrogen (-196°C), etc.

If the drying temperature is -200°C or lower, a refrigerant which is not common must be used which lacks versatility, and it takes a long time to dry for solvent sublimation, efficiency of which is poor. If the drying temperature is 200°C or higher, the ionic bonding reaction on the surface of the coating film proceeds too excessively whereby the surface loses hydrophilicity, and the ability to suppress adhesion of a biological substance(s) is not exhibited. More preferred drying temperature is 10°C to 190°C, 10°C to 180°C, 10°C to 170°C, 10°C to 160°C, 20°C to 180°C, 20°C to 170°C, 20°C to 160°C, 20°C to 150°C and 25°C to 150°C.

After drying, in order to remove impurities, unreacted monomer(s), etc., remained on the coating film, and further, to adjust ion balance of the copolymer in the film, a step of washing with at least one kind of a solvent selected from water and an aqueous solution containing an electrolyte(s) may be carried out. Washing is desirably running water washing or ultrasonic wave washing, etc. The above-mentioned water and the aqueous solution containing an electrolyte(s) may be a material heated, for example, in the range of 40°C to 95°C. The aqueous solution containing an electrolyte(s) is preferably PBS, physiological saline (containing only sodium chloride), Dulbecco's phosphate-buffered saline, Tris buffered physiological saline, HEPES buffered physiological saline and veronal buffered physiological saline, and particularly preferably PBS. After adhesion, the coating film does not elute even when it is washed with water, PBS and alcohol, etc., and still remains firmly adhered to the substrate. Even if the biological substances are adhered to the formed coating film, these can be easily removed by washing, etc., and the surface of the substrate onto which the coating film of the present invention is formed has an ability to suppress the adhesion of the biological substance.

As an application example of the coating film of the present invention, for example, there is a coating film for a filter of an artificial dialyzer, the coating film of the present invention has good adherence of the coating film to a synthetic resin (for example, PES, PS, PSF, etc.) used for a filter and durability after adhesion. The form of the substrate is not particularly limited, and there may be mentioned a substrate, fibers, particles, a gel form, a porous form, etc., and the shape may be a flat plate or a curved surface.

For example, when a coating film for a filter of an artificial dialyzer is to be made, it can be produced by passing a liquid of the composition for forming a coating film according to the present invention through inside a hollow fiber-shaped filter having, for example, a diameter of 0.1 to 500 µm prepared by the above-mentioned materials, and thereafter, subjecting to a drying step and a washing step (washing with hot water (for example, 40°C to 95°C), etc.).

If necessary, a treatment with γ-rays, ethylene oxide, autoclaves, etc., may be carried out for sterilization, in some cases.

A thickness of the coating film of the present invention is preferably 10 to 1,000Å, further preferably 10 to 500Å, and most preferably 20 to 400Å.

The coating film of the present invention has an ability to suppress adhesion of biological substances, so that it can be suitably used as a coating film for a medical substrate. It can be suitably used, for example, a leukocyte removal filter, a blood transfusion filter, a virus removal filter, a microcoagulate removal filter, a module for blood purification, artificial heart, artificial lung, blood circuit, artificial blood vessel, vascular bypass tube, medical tube, artificial valve, cannula, stent, catheter, intracatheter, balloon catheter, guide wires, sutures, indwelling needles, shunts, artificial joints, artificial hip joints, blood bags, blood storage containers, surgical aids, adhesion preventive film, wound covering materials, etc. Here, the module for blood purification refers to a module having a function of circulating blood outside the body to remove waste products and harmful substances in the blood, and examples thereof may be mentioned artificial kidney, a toxin adsorption filter, a column, etc.

Also, the coating film of the present invention is useful as a coating film for cell culture containers such as flasks, dishes, plates, etc., and various kinds of research instruments that suppress adhesion of proteins.

Further, the composition for forming a coating film of the present invention is also useful as a material for cosmetics, a material for contact lens care products, a fiber processing agent for skin care, a material for a diagnostic agent for biochemical research, a blocking agent for suppressing non-specific adsorption in an enzymelinked immunosorbent assay (ELISA) method widely used in clinical diagnostic method or a latex aggregation method, and a stabilizer for stabilizing proteins such as enzymes and antibodies, etc.

Moreover, the coating film of the present invention is also useful as a coating film for toiletries, personal care products, detergents, pharmaceuticals, quasidrugs, fibers, and antifouling materials.

### EXAMPLES

In the following, the present invention is explained in more detail based on Examples and the like, but the present invention is not limited by the following Examples and the like.

### <Measurement method of purity of monomer >

The % by mass (purity) of acid phosphoxypolypropylene glycol monomethacrylate (number of average added mole of propylene oxide: 5) (product name; PPM-5P, available from TOHO Chemical Industry Co., Ltd.) used in the following Synthetic Examples is obtained, after compositional analysis of the phosphorus-containing compound using ³¹P-NMR measurement, by conversion using a molecular weight when the number of added moles of the acid phosphoxypolypropylene glycol monomethacrylate (number of average added mole of propylene oxide: 5) was made 5.

### (³¹P-NMR measurement conditions)

· Apparatus: AVANCE III HD (500MHz)
· Solvent: Deuteroacetone
· Measurement temperature: room temperature (23°C)
· Measurement condition: Non-decoupling method
· Measurement data point: 131072 (128k)
· Pulse width: 90° pulse (14 µsec.)
· Measurement range: -50 to 10 ppm
· Cumulative number: 256 times

### < Measurement method of molecular weight of copolymer>

The weight average molecular weight of the copolymer shown in the following Synthetic Examples is a measurement result by Gel Filtration Chromatography (hereinafter abbreviated to as GFC).

### (Measurement conditions)

· Apparatus: HLC-8320GPC (manufactured by Tosoh Corporation)
· GFC column: TSKgel GMPWXL (7.8mmI.D.×30cm) × 2 to 3 columns
· Eluent: Ionic substance-containing aqueous solution, or a mixed solution with EtOH (ethanol)
· Column temperature: 40°C
· Detector: RI
· Injection concentration: Polymer solid content 0.05 to 0.5%
· Injection amount: 100 µL
· Calibration curve: Cubic approximation curve
· Standard sample: Polyethylene oxide (available from Agilent Technologies) × 10 kinds

### <Synthetic Example 1>

To 2.27 g of acid phosphoxypolypropylene glycol monomethacrylate (number of average added mole of propylene oxide: 5) (product name; PPM-5P, available from TOHO Chemical Industry Co., Ltd., % by mass (purity): 95.6% by mass) was added 16.70 g of ethanol, and the mixture was sufficiently dissolved. Then, 0.80 g of 2-(dimethylamino)ethyl methacrylate (available from Tokyo Chemical Industry Co., Ltd.) and 11.23 g of pure water were added thereto, and after stirring the mixture well, 15.7 mg of dimethyl 1,1'-azobis(1-cyclohexanecarboxylate) (product name; VE-073, available from FUJIFILM Wako Pure Chemical Corporation) was added thereto. A 100 mL flask in which a mixed solution containing the above-mentioned all the materials which became uniform by sufficient stirring had been charged was replaced with nitrogen, and the temperature of the mixture was raised to reflux temperature while stirring (oil bath temperature: set to 95°C). The mixture was heated and stirred by maintaining the above-mentioned circumstance for 24 hours to obtain a copolymer-containing varnish having a solid content of about 10.60% by mass. The weight average molecular weight of the obtained transparent liquid by GFC was about 310,000.

### <Synthetic Example 2>

To a mixed solvent of 10.46 g of pure water and 10.14 g of ethanol were added 0.89 g of 2-methacryloyloxyethyl phosphorylcholine (MPC, available from Tokyo Chemical Industry Co., Ltd.) and 1.37 g of acid phosphoxypolypropylene glycol monomethacrylate (number of average added mole of propylene oxide: 5) (product name; PPM-5P, available from TOHO Chemical Industry Co., Ltd., % by mass (purity): 95.6% by mass), and the mixture was stirred well. Then, 6 mg of dimethyl 1,1'-azobis(1-cyclohexanecarboxylate) (product name; VE-073, available from FUJIFILM Wako Pure Chemical Corporation) was added thereto, and the mixture was sufficiently stirred until which became uniform. A 100 mL flask in which the mixed solution had been charged was replaced with nitrogen, and the temperature of the mixture was raised to 70°C and the mixture was heated and stirred for 7 hours to obtain a copolymer-containing varnish having a solid content of about 9.93% by mass. The weight average molecular weight of the obtained transparent liquid by GFC was about 750,000.

### <Synthetic Example 3>

To a mixed solvent of 8.51 g of pure water and 8.59 g of ethanol were added 1.25 g of 2-methacryloyloxyethyl phosphorylcholine (MPC, available from Tokyo Chemical Industry Co., Ltd.) and 0.84 g of acid phosphoxypolypropylene glycol monomethacrylate (number of average added mole of propylene oxide: 5) (product name; PPM-5P, available from TOHO Chemical Industry Co., Ltd., % by mass (purity): 95.6% by mass), and the mixture was stirred well. Then, 6 mg of dimethyl 1,1'-azobis(1-cyclohexanecarboxylate) (product name; VE-073, available from FUJIFILM Wako Pure Chemical Corporation) was added thereto, and the mixture was sufficiently stirred until which became uniform. A 100 mL flask in which the mixed solution had been charged was replaced with nitrogen, and the temperature of the mixture was raised to 70°C and the mixture was heated and stirred for 7 hours to obtain a copolymer-containing varnish having a solid content of about 11.20% by mass. The weight average molecular weight of the obtained transparent liquid by GFC was about 710,000.

### <Synthetic Example 4>

To a mixed solvent of 8.60 g of pure water and 8.52 g of ethanol were added 1.62 g of 2-methacryloyloxyethyl phosphorylcholine (MPC, available from Tokyo Chemical Industry Co., Ltd.) and 0.28 g of acid phosphoxypolypropylene glycol monomethacrylate (number of average added mole of propylene oxide: 5) (product name; PPM-5P, available from TOHO Chemical Industry Co., Ltd., % by mass (purity): 95.6% by mass), and the mixture was stirred well. Then, 6 mg of dimethyl 1,1'-azobis(1-cyclohexanecarboxylate) (product name; VE-073, available from FUJIFILM Wako Pure Chemical Corporation) was added thereto, and the mixture was sufficiently stirred until which became uniform. A 100 mL flask in which the mixed solution had been charged was replaced with nitrogen, and the temperature of the mixture was raised to 70°C and the mixture was heated and stirred for 7 hours to obtain a copolymer-containing varnish having a solid content of about 10.36% by mass. The weight average molecular weight of the obtained transparent liquid by GFC was about 390,000.

### <Synthetic Examples 5 to 12>

In accordance with Synthetic Example 1, polymers were obtained by using the monomers and the monomer composition with the compositional ratio shown in the table. The molecular weights of the obtained polymers are analyzed in the same manner as in Synthetic Example 1 and the results are shown in Table 1.

Incidentally, PEM5P in Table 1 designates acid phosphoxyethylene glycol monomethacrylate (number of average added mole of ethylene oxide:5) (product name; PEM-5P, available from TOHO Chemical Industry Co., Ltd., % by mass (purity): 87.4% by mass), MHP designates acid phosphoxyhexyl monomethacrylate (product name; MHP, available from TOHO Chemical Industry Co., Ltd., % by mass (purity): 94.2% by mass), DMMA designates 2-(dimethylamino)ethyl methacrylate (available from Tokyo Chemical Industry Co., Ltd.), DEMA designates 2-(diethylamino)ethyl methacrylate (available from Tokyo Chemical Industry Co., Ltd.), and MACC designates about 80% aqueous solution of methacryloyl choline chloride (available from Tokyo Chemical Industry Co., Ltd.), respectively.

In addition, % by mass (purity) of PEM5P and MHP was analyzed by the same manner as that of PPM5P described in Synthetic Examples 1 to 4. With regard to PEM5P, it was obtained by conversion using the molecular weight when the number of average added mole of ethylene oxide was 5.

**[Table 1]**

| Synthetic Example | Monomer composition (mol%) | | | | | | | Solid content (% by mass) | Molecular weight (Mw) |
|---|---|---|---|---|---|---|---|---|---|
| | PPM5P | PEM5P | MHP | DMMA | MPC | DEMA | MACC | | |
| 1 | 50 | | | 50 | | | | 10.60 | 310000 |
| 2 | 50 | | | | 50 | | | 9. 93 | 750000 |
| 3 | 30 | | | | 70 | | | 11. 20 | 710000 |
| 4 | 10 | | | | 90 | | | 10. 36 | 390000 |
| 5 | 70 | | | 30 | | | | 10. 76 | 280000 |
| 6 | 30 | | | 70 | | | | 10. 07 | 250000 |
| 7 | 10 | | | 90 | | | | 7. 05 | 180000 |
| 8 | 50 | | | | | 50 | | 10. 44 | 210000 |
| 9 | 70 | | | | | | 30 | 9. 87 | 190000 |
| 10 | 50 | | | | | | 50 | 10. 66 | 140000 |
| 11 | | 50 | | 50 | | | | 9. 88 | 100000 |
| 12 | | | 50 | | | | 50 | 10. 40 | 450000 |

### <Comparative Synthetic Example 1>

The % by mass of the phosphorus-containing compound of acid phosphoxyethyl methacrylate mentioned below is a value obtained by ³¹P-NMR measurement described in WO 2016/093293A1.

9.22 g of ethanol was added to 1.06 g of acid phosphoxyethyl methacrylate (product name; Phosmer M, available from UniChemical Co., non-volatile content by the evaporation to dryness method at 100°C for 1 hour: 91.8%, % by mass of each component: a mixture of acid phosphoxyethyl methacrylate (44.2% by mass), bis[2-(methacryloyloxy)ethyl] phosphate (28.6% by mass), and other substances (27.2% by mass)), and the mixture was sufficiently dissolved. Then, 0.72 g of 2-(dimethylamino)ethyl methacrylate (available from Tokyo Chemical Industry Co., Ltd.) and 6.00 g of pure water were added thereto, and after stirring the mixture well, 8.6 mg of dimethyl 1,1'-azobis(1-cyclohexanecarboxylate) (product name; VE-073, available from FUJIFILM Wako Pure Chemical Corporation) was added thereto. A 50 mL flask in which a mixed solution containing the above-mentioned all the materials which became uniform by sufficient stirring had been charged was replaced with nitrogen, and the temperature of the mixture was raised to reflux temperature while stirring (oil bath temperature: set to 95°C). The mixture was heated and stirred by maintaining the above-mentioned circumstance but white precipitates were generated within 1 hour.

### <Comparative Synthetic Example 2>

To a mixed solvent of 7.26 g of pure water and 7.24 g of ethanol were added 0.90 g of 2-methacryloyloxyethyl phosphorylcholine (MPC, available from Tokyo Chemical Industry Co., Ltd.) and 0.73 g of acid phosphoxyethyl methacrylate (product name; Phosmer M, available from UniChemical Co., non-volatile content by the evaporation to dryness method at 100°C for 1 hour: 91.8%, % by mass of each component: a mixture of acid phosphoxyethyl methacrylate (44.2% by mass), bis[2-(methacryloyloxy)ethyl] phosphate (28.6% by mass), and other substances (27.2% by mass)), and the mixture was stirred well. Then, 6 mg of dimethyl 1,1'-azobis(1-cyclohexanecarboxylate) (product name; VE-073, available from FUJIFILM Wako Pure Chemical Corporation) was added thereto, and the mixture was sufficiently stirred until which became uniform. A 50 mL flask in which a mixed solution had been charged was replaced with nitrogen, and after raising the temperature to 70°C, the mixture was heated and stirred by maintaining this circumstance but white precipitates were generated within 3 hour.

### <Example 1>

To 0.80 g of the copolymer-containing varnish obtained in the above-mentioned Synthetic Example 1 were added 5.05 g of pure water, 1.94 g of ethanol and 0.20 g of 0.1 mol/L hydrochloric acid (1N hydrochloric acid (available from Kanto Chemical Co., Inc.) was diluted 10-fold with pure water and used) and the mixture was sufficiently stirred to prepare a composition for forming a coating film. The pH was 4.6. The obtained composition for forming a coating film was spin-coated on an HMDS-treated silicon wafer with 1,500 rpm/60 sec, and as a drying step, it was dried in an oven at 50°C for 24 hours. Thereafter, it was sufficiently washed with phosphate buffer solution (hereinafter abbreviated to as PBS) and then dried in an oven at 50°C for 1 hour to obtain a coating film on the HMDS-treated silicon wafer. When the film thickness of the coating film on the HMDS-treated silicon wafer was confirmed by a spectroscopic ellipsometer, it was 3.9 nm.

### <Example 2>

To 0.80 g of the copolymer-containing varnish obtained in the above-mentioned Synthetic Example 1 were added 5.26 g of pure water and 1.94 g of ethanol and the mixture was sufficiently stirred to prepare a composition for forming a coating film. The pH was 5.9. The obtained composition for forming a coating film was spin-coated on an HMDS-treated silicon wafer with 1,500 rpm/60 sec, and as a drying step, it was dried in an oven at 50°C for 24 hours. Thereafter, it was sufficiently washed with phosphate buffer solution (hereinafter abbreviated to as PBS) and then dried in an oven at 50°C for 1 hour to obtain a coating film on the HMDS-treated silicon wafer. When the film thickness of the coating film on the HMDS-treated silicon wafer was confirmed by a spectroscopic ellipsometer, it was 4.0 nm.

### <Example 3>

To 0.80 g of the copolymer-containing varnish obtained in the above-mentioned Synthetic Example 1 were added 4.84 g of pure water and 1.95 g of ethanol and the mixture was sufficiently stirred, then, 0.26 g of a 0.1 mol/L sodium hydroxide aqueous solution (1N sodium hydroxide (available from Kanto Chemical Co., Inc.) was diluted 10-fold with pure water and used) was added thereto and the mixture was sufficiently stirred to prepare a composition for forming a coating film. The pH was 7.0. The obtained composition for forming a coating film was spin-coated on an HMDS-treated silicon wafer with 1,500 rpm/60 sec, and as a drying step, it was dried in an oven at 50°C for 24 hours. Thereafter, it was sufficiently washed with phosphate buffer solution (hereinafter abbreviated to as PBS) and then dried in an oven at 50°C for 1 hour to obtain a coating film on the HMDS-treated silicon wafer. When the film thickness of the coating film on the HMDS-treated silicon wafer was confirmed by a spectroscopic ellipsometer, it was 3.9 nm.

### <Example 4>

To 2.00 g of the copolymer-containing varnish obtained in the above-mentioned Synthetic Example 2 were added 9.00 g of pure water and 9.00 g of ethanol and the mixture was sufficiently stirred to prepare a composition for forming a coating film. The pH was 2.8. The obtained composition for forming a coating film was spin-coated on an HMDS-treated silicon wafer with 1,500 rpm/60 sec, and as a drying step, it was dried in an oven at 50°C for 24 hours. Thereafter, it was sufficiently washed with PBS and then dried in an oven at 50°C for 1 hour to obtain a coating film on the HMDS-treated silicon wafer. When the film thickness of the coating film on the HMDS-treated silicon wafer was confirmed by a spectroscopic ellipsometer, it was 3.9 nm.

### <Example 5>

To 2.00 g of the copolymer-containing varnish obtained in the above-mentioned Synthetic Example 3 were added 9.00 g of pure water and 9.00 g of ethanol and the mixture was sufficiently stirred to prepare a composition for forming a coating film. The pH was 3.1. The obtained composition for forming a coating film was spin-coated on an HMDS-treated silicon wafer with 1,500 rpm/60 sec, and as a drying step, it was dried in an oven at 50°C for 24 hours. Thereafter, it was sufficiently washed with PBS and then dried in an oven at 50°C for 1 hour to obtain a coating film on the HMDS-treated silicon wafer. When the film thickness of the coating film on the HMDS-treated silicon wafer was confirmed by a spectroscopic ellipsometer, it was 3.5 nm.

### <Example 6>

To 2.00 g of the copolymer-containing varnish obtained in the above-mentioned Synthetic Example 4 were added 9.00 g of pure water and 9.00 g of ethanol and the mixture was sufficiently stirred to prepare a composition for forming a coating film. The pH was 3.3. The obtained composition for forming a coating film was spin-coated on an HMDS-treated silicon wafer with 1,500 rpm/60 sec, and as a drying step, it was dried in an oven at 50°C for 24 hours. Thereafter, it was sufficiently washed with PBS and then dried in an oven at 50°C for 1 hour to obtain a coating film on the HMDS-treated silicon wafer. When the film thickness of the coating film on the HMDS-treated silicon wafer was confirmed by a spectroscopic ellipsometer, it was 3.1 nm.

### <Example 7>

To 1.00 g of the copolymer-containing varnish obtained in the above-mentioned Synthetic Example 5 were added 2.60 g of pure water and 6.40 g of ethanol and the mixture was sufficiently stirred to prepare a composition for forming a coating film. The pH was 3.6. The obtained composition for forming a coating film was spin-coated on an HMDS-treated silicon wafer with 1,500 rpm/60 sec, and as a drying step, it was dried in an oven at 50°C for 24 hours. Thereafter, it was sufficiently washed with PBS and then dried in an oven at 50°C for 1 hour to obtain a coating film on the HMDS-treated silicon wafer. When the film thickness of the coating film on the HMDS-treated silicon wafer was confirmed by a spectroscopic ellipsometer, it was 3.5 nm.

### <Example 8>

To 1.00 g of the copolymer-containing varnish obtained in the above-mentioned Synthetic Example 8 were added 2.60 g of pure water and 6.40 g of ethanol and the mixture was sufficiently stirred to prepare a composition for forming a coating film. The pH was 6.8. The obtained composition for forming a coating film was spin-coated on an HMDS-treated silicon wafer with 1,500 rpm/60 sec, and as a drying step, it was dried in an oven at 50°C for 24 hours. Thereafter, it was sufficiently washed with PBS and then dried in an oven at 50°C for 1 hour to obtain a coating film on the HMDS-treated silicon wafer. When the film thickness of the coating film on the HMDS-treated silicon wafer was confirmed by a spectroscopic ellipsometer, it was 3.8 nm.

### <Example 9>

To 1.00 g of the copolymer-containing varnish obtained in the above-mentioned Synthetic Example 10 were added 2.50 g of pure water and 6.40 g of ethanol and the mixture was sufficiently stirred, then, 0.11 g of a 0.1 mol/L sodium hydroxide aqueous solution (1N sodium hydroxide (available from Kanto Chemical Co., Inc.) was diluted 10-fold with pure water and used) was added thereto and the mixture was sufficiently stirred to prepare a composition for forming a coating film. The pH was 4.2. The obtained composition for forming a coating film was spin-coated on an HMDS-treated silicon wafer with 1,500 rpm/60 sec, and as a drying step, it was dried in an oven at 50°C for 24 hours. Thereafter, it was sufficiently washed with PBS and then dried in an oven at 50°C for 1 hour to obtain a coating film on the HMDS-treated silicon wafer. When the film thickness of the coating film on the HMDS-treated silicon wafer was confirmed by a spectroscopic ellipsometer, it was 3.7 nm.

### <Reference Example 1>

49.75 g of ethanol was added to 0.25 g of Lipidure-CM5206 (available from NOF CORPORATION) and the mixture was sufficiently stirred to prepare a composition for forming a coating film. The obtained composition for forming a coating film was spin-coated on an HMDS-treated silicon wafer with 1,500 rpm/60 sec, and as a drying step, it was dried in an oven at 50°C for 24 hours. Thereafter, it was sufficiently washed with PBS and then dried in an oven at 50°C for 1 hour to obtain a coating film on the HMDS-treated silicon wafer. When the film thickness of the coating film on the HMDS-treated silicon wafer was confirmed by a spectroscopic ellipsometer, it was 25.4 nm.

### <Test Example 1: Cell adhesion inhibitory effect>

### (Preparation of coating plate)

The compositions for forming a coating film prepared in Examples 1 to 3, 8 and 9 were each added to separate wells of a 96-well cell culture plate (#351172 available from Corning Co., volume: 0.37 mL, made of polystyrene) so as to be 200 µL/well, and after allowing to stand at room temperature for 1 hour, excess varnish was removed. It was dried using an oven at 50°C for 24 hours. Thereafter, each well subjected to coating was washed with 250 µL of pure water each three times, and after drying using an oven at 50°C for 1 hour, used for the test. As a positive control, a material in which the composition for forming a coating film prepared in Reference Example 1 was coated onto a well of a 96-well cell culture plate (#351172 available from Corning Co., volume: 0.37 mL, made of polystyrene) in the same manner as mentioned above was used. As a negative control, a 96-well cell culture plate (#351172 available from Corning Co., volume: 0.37 mL, made of polystyrene) to which no coating was applied was used.

### (Preparation of cells)

As the cells, mouse embryonic fibroblasts (available from DS Pharma Biomedical Co., Ltd.) were used. The medium used for culture of the cells was a BME medium (available from Thermo Fisher Scientific Co., Ltd.) containing 10% FBS (available from Sigma-Aldrich Corporation) and an L-glutamine-penicillin-streptomycin stabilizing solution (available from Thermo Fisher Scientific Co., Ltd.). The cells were statically cultured for 2 days or longer using a schale (petri dish) (10 mL of medium) having a diameter of 10 cm while in a state of maintaining a 5% carbon dioxide concentration in a 37°C/CO₂ incubator. Subsequently, the present cells were washed with 5 mL of PBS, then, 1 mL of 0.25 w/v% trypsin-1 mmol/L EDTA solution (available from FUJIFILM Wako Pure Chemical Corporation) was added thereto, and the cells were detached and suspended in 10 mL of the above-mentioned medium, respectively. This suspension was centrifuged (model number LC-200 manufactured by Tommy Seiko Co., Ltd., 1,000 rpm/3 minutes, room temperature), then, the supernatant was removed and the above-mentioned medium was added to prepare a cell suspension.

### (Cell adhesion test)

150 µL of each cell suspension was added to each well, the positive control and the negative control of the plates prepared as mentioned above so as to be 1 × 10⁴ cells/well. Thereafter, the samples were allowed to stand in a CO₂ incubator at 37°C for 3 days in the state of maintaining a 5% carbon dioxide concentration.

### (Observation of cell adhesion)

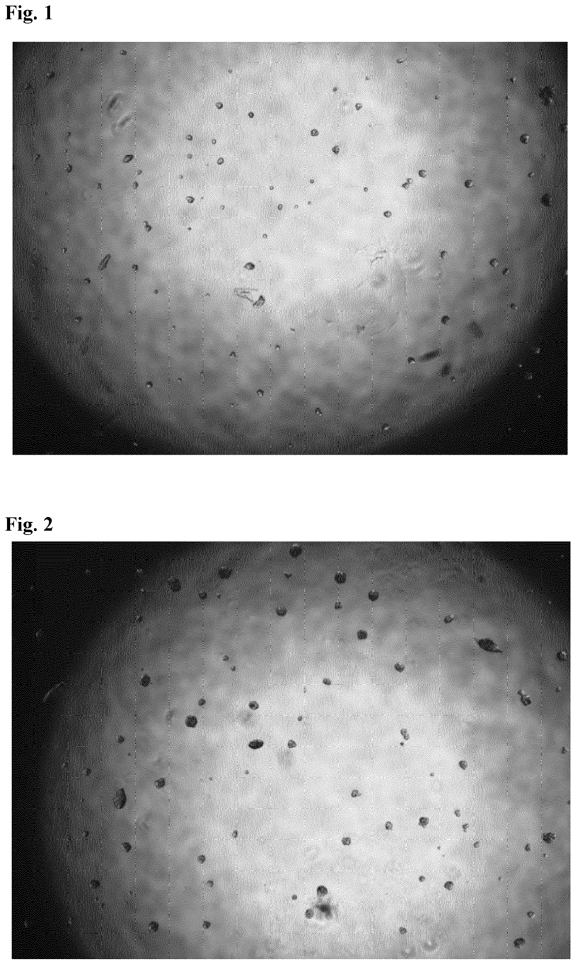

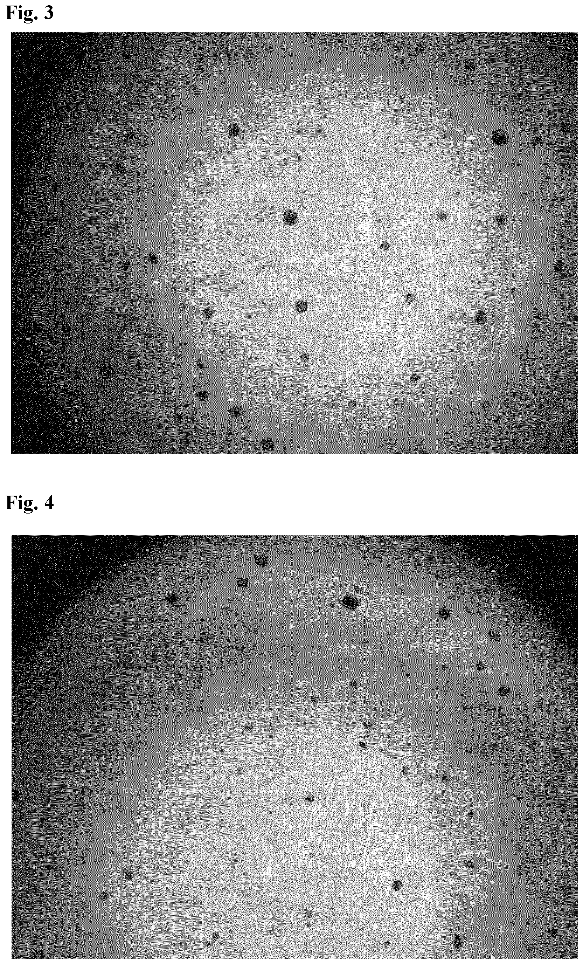

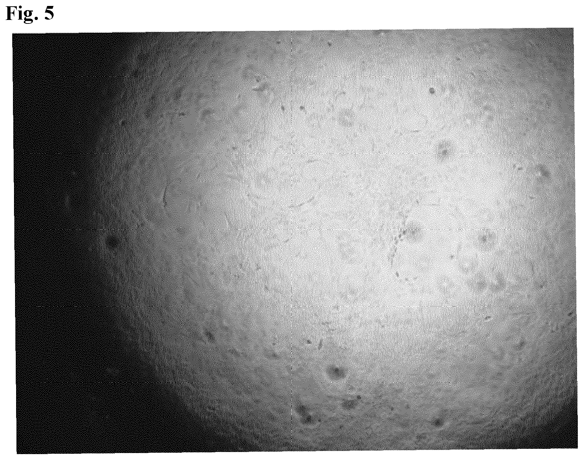

Three days after the culture, adhesion of the cells to each well, the positive control and the negative control of the plates prepared as mentioned above were compared based on observation (magnification: 4-fold) with an inverted microscope (CKX31 manufactured by Olympus Corporation). In each well of the plates coated by coating agents (Reference Example 1) of Example 1 to 3, 8, 9 and the positive control, no adhesion of the cells was observed, but in the well of the negative control, adhesion of the cells was observed. The results of Examples 1 to 3, the positive control and the negative control are shown in FIGs. 1 to 5, respectively.

### <Test Example 2: Protein adsorption suppressing effect>

### (Preparation of QCM sensor (PS))

An Au-deposited quartz oscillator (Q-Sense,QSX301) was washed at 50 mA for 3 min by using a soft etching apparatus (SEDE-GE manufactured by MEIWAFOSIS Co., Ltd.), and immediately thereafter immersed in a solution in which 0.0772 g of 2-aminoethanethiol (available from Tokyo Chemical Industry Co., Ltd.) had been dissolved in 1,000 mL of ethanol for 24 hours. After washing the surface of the sensor with ethanol, it was naturally dried, and a varnish in which 1.00 g of polystyrene (available from Sigma-Aldrich Co.) had been dissolved in 99.00 g of toluene was spin coated onto a side of a membrane sensor with 3,500 rpm/30 sec and dried at 150°C for 1 minutes to prepare a QCM sensor (PS).

### (Preparation QCM sensor (SiO₂))

An SiO₂-deposited quartz oscillator (Q-Sence, QSX303) was washed at 50 mA for 3 min by using a soft etching apparatus (SEDE-GE manufactured by MEIWAFOSIS Co., Ltd.), and used as such.

### (Preparation of surface-treated QCM sensor (PS) and surface-treated QCM sensor (SiO₂))

The compositions for forming a coating film obtained in Examples 1 to 4 and 7 to 9 and Reference Example 1 were each spin-coated to a QCM sensor (PS) at 3,500 rpm/30 sec, and as a drying step, baked in an oven at 50°C for 24 hours. Thereafter, as a washing step, the excess composition for forming a coating film was washed each twice with PBS and pure water to obtain surface-treated QCM sensors (PS) (Substrates Nos. 1 to 5 and 11 to 13). Similarly, the compositions for forming a coating film obtained in Examples 4 to 6 were each spin-coated on a QCM sensor (SiO₂) at 3,500 rpm/30 sec, and as a drying step, it was baked in an oven at 50°C for 24 hours. Thereafter, as a washing step, the excess composition for forming a coating film was washed each twice with PBS and pure water to obtain surface-treated QCM sensors (SiO₂) (Substrates Nos. 7 to 9). As a positive control, a surface-treated QCM sensor (PS) (Substrate No. 5) to which the composition for forming a coating film obtained in Reference Example 1 was coated was used. As a negative control, a QCM sensor (PS) (Substrate No. 6) and a QCM sensor (SiO₂) (Substrate No. 10) were used.

### (Protein adsorption experiment)

The surface-treated QCM sensors (PS) (Substrates Nos. 1 to 5, 11 to 13) and the surface-treated QCM sensors (SiO₂) (Substrates Nos. 7 to 9) prepared by the above-mentioned method using the compositions for forming a coating film were each attached to a dissipation type quartz crystal microbalance QCM-D (E4, Q-Sence), and PBS was flown until a stable baseline was established in which change in the frequency was 1 Hz or less in 1 hour. Next, PBS was flown for about 10 minutes with the frequency of the stable base line as 0 Hz. Subsequently, a 100 µg/mL PBS solution of human serum-derived γ-globulin was flown for about 30 minutes, and then, after flowing PBS again for about 20 minutes, the shift (Δf) of the adsorption-induced frequency of 9th-order overtone was read. For analysis, Q-Tools (Q-Sence) are used, and the value in which the shift (Δf) of the adsorption-induced frequency is converted to the mass (ng/cm²) per unit area of the shift (Δf) of the adsorption-induced frequency explained by the Sauerbrey equation is shown in the following Table 2 as the attached amount of the biological substance. Substrates Nos. 1 to 4, Substrates Nos. 7 to 9 and Substrates Nos. 11 to 13 in which the compositions for forming a coating film of Examples 1 to 9 according to the present invention were used as a coating showed low protein adsorption amount as compared with Substrates Nos. 6 and 10 having no coating.

**[Table 2]**

| Substrate No. | QCM sensor | Coating agent | Protein adhered amount (ng/cm²) |
|---|---|---|---|
| 1 | PS | Example 1 | 21 |
| 2 | PS | Example 2 | 106 |
| 3 | PS | Example 3 | 20 |
| 4 | PS | Example 4 | 333 |
| 5 | PS | Reference Example 1 | 29 |
| 6 | PS | None | 946 |
| 7 | SiO₂ | Example 4 | 5 |
| 8 | SiO₂ | Example 5 | 19 |
| 9 | SiO₂ | Example 6 | 7 |
| 10 | SiO₂ | None | 323 |
| 11 | PS | Example 7 | 130 |
| 12 | PS | Example 8 | 48 |
| 13 | PS | Example 9 | 235 |

### UTILIZABILITY IN INDUSTRY

According to the present invention, it can provide a method for producing a copolymer-containing varnish which does not cause the problem of gelation, a method for producing a composition for forming a coating film, a method for producing a coating film, preferably a method for producing a coating film compatible with biomaterials (for example, having an adhesion suppressing ability to biomaterials and a cell culture promoting property).

The coating film of the present invention has an ability to suppress adhesion of biological substances, so that it can be suitably used as a coating film for a medical substrate. It can be suitably used, for example, leukocyte removal filter, blood transfusion filter, virus removal filter, microcoagulate removal filter, a module for blood purification, artificial heart, artificial lung, blood circuit, artificial blood vessel, vascular bypass tube, medical tube, artificial valve, cannula, stent, catheter, intracatheter, balloon catheter, guide wires, sutures, indwelling needles, shunts, artificial joints, artificial hip joints, blood bags, blood storage containers, surgical aids, adhesion preventive film, wound covering materials, etc. Here, the module for blood purification refers to a module having a function of circulating blood outside the body to remove waste products and harmful substances in the blood, and examples thereof may be mentioned artificial kidney, a toxin adsorption filter, a column, etc.

Also, the coating film of the present invention is useful as a coating film for cell culture containers such as flasks, dishes, plates, etc., and various kinds of research instruments that suppress adhesion of proteins.

Further, the composition for forming a coating film of the present invention is also useful as a material for cosmetics, a material for contact lens care products, a fiber processing agent for skin care, a material for a diagnostic agent for biochemical research, a blocking agent for suppressing non-specific adsorption in an enzymelinked immunosorbent assay (ELISA) method widely used in clinical diagnostic method or a latex aggregation method, and a stabilizer for stabilizing proteins such as enzymes and antibodies, etc.

Moreover, the coating film of the present invention is also useful as a coating film for toiletries, personal care products, detergents, pharmaceuticals, quasidrugs, fibers, and antifouling materials.

## Claims

1. A method for producing a copolymer-containing varnish which comprises a step of copolymerizing a monomer mixture containing compounds represented by the following formula (1) and the formula (2): 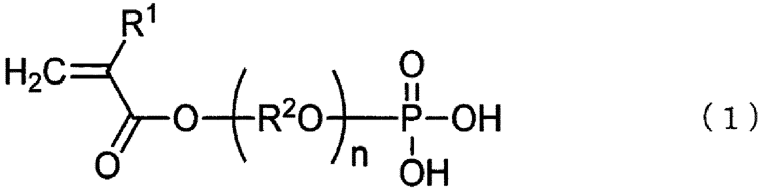 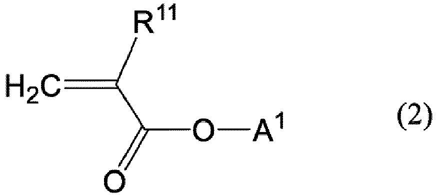
in the formula (1), R¹ represents a hydrogen atom or a methyl group, R² represents an alkylene group having 1 to 6 carbon atoms, n represents an integer of 1 to 30, in the formula (2), R¹¹ represents a hydrogen atom or a methyl group, and A¹ represents a monovalent organic group having a cationic property, in a solvent for polymerization, in the presence of a radical polymerization initiator,
wherein % by mass of the compound represented by the formula (1) in a phosphorus-containing compound contained in the monomer mixture is 70% by mass or more.

2. The method for producing a copolymer-containing varnish according to Claim 1, wherein the A¹ contains the structure represented by the following formula (2-1): 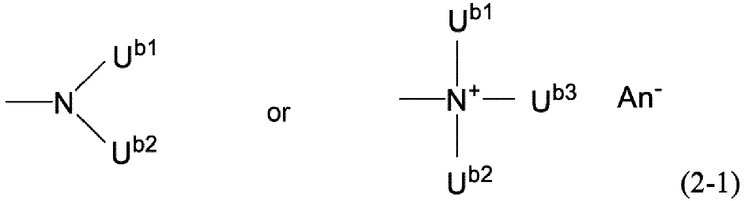 U^{b1}, U^{b2} and U^{b3} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion.

3. The method for producing a copolymer-containing varnish according to Claim 1, wherein the A¹ is represented by the following formula (2-2): 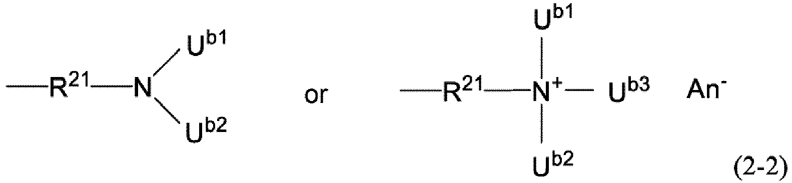 R²¹ represents an alkylene group having 1 to 6 carbon atoms which may be interrupted by a phosphodiester bond, U^{b1}, U^{b2} and U^{b3} each independently represent a hydrogen atom or a linear or branched alkyl group having 1 to 5 carbon atoms, and An⁻ represents an anion selected from the group consisting of a halide ion, an inorganic acid ion, a hydroxide ion and an isothiocyanate ion.

4. A method for producing a composition for forming a coating film which comprises a step of copolymerizing a monomer mixture containing compounds represented by the following formula (1) and the formula (2): 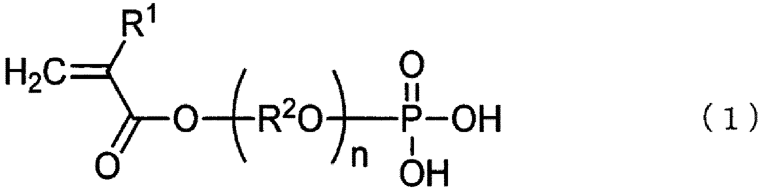 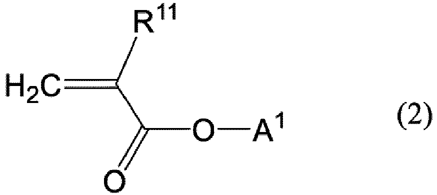
in the formula (1), R¹ represents a hydrogen atom or a methyl group, R² represents an alkylene group having 1 to 6 carbon atoms, n represents an integer of 1 to 30, in the formula (2), R¹¹ represents a hydrogen atom or a methyl group, and A¹ represents a monovalent organic group having a cationic property, in a solvent for polymerization, in the presence of a radical polymerization initiator, and then, a step of adding a solvent,
wherein % by mass of the compound represented by the formula (1) in a phosphorus-containing compound contained in the monomer mixture is 70% by mass or more.

5. The method for producing a composition for forming a coating film according to Claim 4, which is compatible with biomaterials.

6. The method for producing a coating film, which comprises a step of coating the composition for forming a coating film according to Claim 4 or 5 to a substrate.
